# EUROPEAN PATENT SPECIFICATION

(11) **EP 4 146 152 B1**
(45) Date of publication and mention of the grant of the patent: **15.11.2023**
(21) Application number: 21721943.5
(22) Date of filing: 30.04.2021
(51) Int. Cl.: A61K 8/25, A61K 8/26, A61K 8/73, A61K 8/86, A61K 8/89, A61Q 15/00, A61K 8/34

(54) **AN ANTIPERSPIRANT COMPOSITION**
SCHWEISSHEMMENDE ZUSAMMENSETZUNG
COMPOSITION ANTITRANSPIRATION

(30) Priority: 07.05.2020 IN 202021019493; 30.06.2020 EP 20183137
(43) Date of publication of application: 15.03.2023
(73) Proprietor: Unilever IP Holdings B.V., 3013 AL Rotterdam (NL); Unilever Global IP Limited, Wirral, Merseyside CH62 4ZD (GB)
(72) Inventor: ALLAHBASH, Shahin, BANAGALORE 560066 Karnataka (IN); BARNE, Sameer, Keshav, BANAGALORE 560066 Karnataka (IN)
(74) Representative: Whaley, Christopher
(86) International application number: PCT/EP2021/061420
(87) International publication number: WO 2021/224133

(56) References cited:
- WO-A1-93/24105
- US-A- 4 963 591
- US-B1- 6 387 356

## Description

### Field of the invention

The present invention is in the field of compositions comprising antiperspirant actives, particularly cosmetic compositions. The present invention more particularly relates to compositions which do not contain metal based antiperspirant actives yet exhibit efficacy similar to or better than that of well known aluminium based actives.

### Background of the invention

The present invention relates to compositions, such as those that contain antiperspirant actives. These actives are added to compositions to reduce perspiration on application to the surface of the body, particularly to the underarm regions of the human body viz. the axilla. Antiperspirant actives are typically astringent metal salts such as those of aluminium or zirconium salts. Antiperspirant actives are usually incorporated in compositions at low pH, in the range of 2 to 7. There has been a thrust to develop anti perspirant actives which are less astringent and the approach has been to look for actives that do not contain metal like aluminium. The present inventors with their extensive research in the field of such actives have hit upon a formulation which is a combination of a specific film forming polymer of modified cellulose, an organic solvent and a plasticizer which is found to have similar or better efficacy as compared to that of well-established aluminium based actives.

Certain antiperspirant compositions having low or no amount of metal actives been reported e.g. EP0440387A1 (Unilever, 1991) discloses an antiperspirant composition which is suitable for topical application to human skin, comprising: (i) from 4 to 95 percent by weight of hydrophobically-treated clay; and (ii) from 96 to 5 percent by weight of a carrier medium comprising an alkanol having from 1 to 4 carbon atoms; the composition containing not more than 0.2 percent by weight of aluminium.

FR3014314 (L'Oreal, 2013) discloses a cosmetic composition, comprising a cosmetically acceptable medium, containing a fatty phase and an aqueous phase, said aqueous phase comprising one or more monoalcohol (s) comprising from 2 to 8 carbon atoms and said fatty phase comprising at least one lipophilic film-forming polymer said lipophilic film-forming polymer being neither a vinyl polymer having at least one unit derived from carbosiloxane dendrimer, nor a silicone resin MQ, wherein the amount of monoalcohol (s) is from 8% to 40% by weight relative to the total weight of said composition.

US6387356B1 (Colgate-Palmolive, 2002) discloses antiperspirant compositions with a film forming cellulose. To this aim are used water-insoluble cellulose esters such as acetate propionate cellulose and acetate butyrate cellulose polymers, that are soluble in alcohol and soluble in ethanol which is the main solvent. The Hansen solubility parameter of the solvent is chosen between 20.5-31.0 MPa^{0.5} and plasticisers (such as phthalate) are comprised in the products. The film-forming antiperspirant compositions can be used without additional antiperspirant active such as astringent aluminium salts.

While non-metal based antiperspirant compositions have been report, there is always a need for such compositions that exhibit better and better efficacy. It is thus an object of the present invention to provide for an antiperspirant composition which exhibits high antiperspirant efficacy while having reduced astringency.

### Summary of the invention

The first aspect of the invention relates to an anti-perspirant composition comprising:
(i) a film forming polymer selected from modified cellulose having a solubility in water of less than 1 wt%;
(ii) an organic solvent having a Hansen Solubility Parameter in the range of 17 to 28 MPa^{0.5} ; and
(iii) a plasticizer;

wherein the modified cellulose is a C1 to C7 alkyl cellulose; and
wherein the plasticizer is selected from one or more of triethyl citrate,
polypropylene oxide (with Tg lower than 25°C), glycerol monostearate,
stearic acid, cetyl alcohol, triethyl phosphate, or mineral oil.

It is preferred that the composition additionally comprises a filler selected from MQ resin, silica or clays.

According to another aspect of the present invention there is provided a non-therapeutic method of reducing perspiration comprising the step of applying a composition as claimed in the first aspect on to the desired skin surface.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated.

Numerical ranges expressed in the format "from x to y" are understood to include x and y. When for a specific feature multiple preferred ranges are described in the format "from x to y", it is understood that all ranges combining the different endpoints are also contemplated.

The compositions of the invention are typically "personal care compositions", suitable for cosmetic use as detailed below. Further, use of the compositions of the invention is cosmetic, non-therapeutic use.

In some embodiments of the present invention, the compositions may be used for the therapeutic treatment of hyperhidrosis (extreme sweating).

By "An Antiperspirant Composition" as used herein, is meant to include a composition for topical application to the skin of mammals, especially humans. Such a composition is preferably of the leave-on type. By a leave-on composition is meant a composition that is applied to the desired skin surface and left on for a period of time (say from one minute to 24 hours) after which it may be wiped or rinsed off with water, usually during the regular course of personal washing. The composition may also be formulated into a product which is applied to a human body for improving the appearance, cleansing, odor control or general aesthetics. The composition of the present invention can be an anhydrous composition which is preferred to be delivered from an aerosol / propellant containing composition. Alternately it may also be delivered in the form of a gel or in stick form or may be delivered through a roll-on device. It is especially useful for delivering compositions to the axilla of an individual for anti-perspirancy benefits. "Skin" as used herein is meant to include skin on any part of the body where one may sweat (e.g., neck, chest, back, arms, underarms, hands, legs, buttocks and scalp) especially the underarms.

The present invention is directed to an antiperspirant composition comprising a film forming polymer selected from modified cellulose having a solubility in water of less than 1 wt%; an organic solvent having a Hansen Solubility Parameter and in the range of 18 to 28 MPa^{0.5}; and a plasticizer, as previously defined.

It is preferred that the Hansen solubility parameter of the organic solvent is close to that of the film forming polymer.

The film forming polymer is an alkyl cellulose having 1 to 7 preferably 1 to 4 carbon atoms in the alkyl chain. Thus, the film forming polymers are C1 to C7, more preferably C1 to C4 alkyl cellulose. The most preferred alkyl cellulose is ethyl cellulose. Ethyl cellulose is available as Ethocel (Dow chemicals) and as Aqualon (Ashland).

It is preferred that the ethyl cellulose for use in the invention has a degree of substitution (DS) in the range of 1.7 to 2.8, preferably in the range of 1.8 to 2.4.

It is preferred that the film forming polymer is included in 1.5 to 25%, preferably 2.5 to 20%, further more preferably from 5 to 15% by weight of the composition.

The composition comprises an organic solvent having a Hansen Solubility Parameter in the range of 17 to 28 MPa^{1/2}, preferably in the range of 18 to 25 MPa^{1/2}.

Hansen solubility is used to determine the degree of compatibility and miscibility of two materials. For example, we can judge the solubility and compatibility of a solvent and polymer to form a homogeneous mixture. The Hansen solubility parameter HSP is the sum of the δD for Dispersion (van der Waals), δP for Polarity (related to dipole moment) and δH for hydrogen bonding forces. The closer the HSP value of two materials, the higher is the compatibility of the two materials to blend with each other in solution. The Hansen partial solubility parameters of the organic solvent preferred for use in the present invention are in the ranges: non-polar (δd) from 16.6 to 17.3, polar (δp) from 6.6 to 8.3, and hydrogen bond (δh) from 8.5 to 10.0.

Examples of suitable organic solvents that could be used are ethanol, toluene, glycol ethers, or isododecane, preferably ethanol and glycol ethers. most preferably ethanol. Dowanol (which is a glycol ether) is also a preferred solvent for use in the present invention.

Dowanol is a glycol ether. It is the tradename for the compound with the chemical formula Propylene Glycol n-Propyl Ether C3H7OCH2CH(CH3)OH. The chemical structure is as given below:

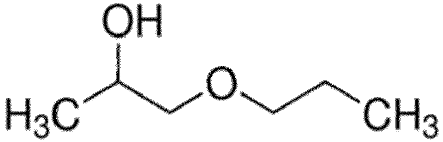

It is preferred that the solvent is included in 40 to 97%, preferably 75 to 90% by weight of the composition. It is further preferred that the film forming polymer is included in 1.5 to 25% by weight of the solvent..

The composition comprises a plasticizer. Plasticizer as per this invention is a material that is included in the composition to reduce the brittleness and increase the flexibility of the film formed as a consequence of applying the composition on a desirable substrate e.g. the skin. In other words, they increase the elasticity of the film and make them softer. Plasticizer for use in the present invention is selected from one or more of polypropylene oxide, PPO in short, having a glass transition temperature T_{g} of less than 25 °C, triethyl phosphate, mineral oils, glycerol Monostearate, stearic acid, triethyl citrate, or cetyl alcohol.

The most preferred plasticizer is selected from triethyl citrate, PPO (with Tg lower than 25°C), glycerol monostearate, stearic acid, cetyl alcohol, triethyl phosphate, mineral oil.

An especially preferred plasticizer is polypropylene oxide (PPO) which is also known as poly propylene glycol (PPG). PPO for use in the present invention preferably has a glass transition temperature (Tg) of less than 25 °C. The glass transition temperature of a polymer is the temperature at which polymers change from a hard and rigid state to a more flexible and supple state. Tg occurs in a temperature range over which the mobility of the polymer chains increases significantly. The right plasticizer alters the property of the polymer film to render it the desired flexibility and opacity. Polypropylene glycol is a polymer derived from monomer propylene glycol. The term polypropylene glycol or PPG is used for low to medium molecular weight polymer when the nature of the endgroup, which is usually a hydroxyl group gives significant contribution to the overall property of the polymer. The PPG for use in the present invention is preferably selected from PPG P1200 or PPG P2000 which are available from Dow chemicals

The composition preferably comprises a particulate filler selected from MQ resin, clay, mica, titanium dioxide, zinc oxide, calcium carbonate or silica, more preferably MQ resin, silica or clay and most preferably MQ resin. MQ resin is a branched, cage like oligo-silicone. It represents a broad range of hydrolytic condensation products of monofunctional silane (M) and tetrafunctional silane (Q), were synthesized by reaction of water glass with hexamethyldisiloxane. For all MQ copolymers, their high-molecular and middle fractions are solid substances, and the low-molecular fraction are liquid in nature. Glass transition temperature of high molecular mass fractions are either above the decomposition temperature or above 300 ∘C, while the average molecular mass of other fractions has glass transition temperature in the range of 80 to 200 °C, For liquid resins, with low molecular mass Tg is in the range of -40 ∘C to -10 °C. They have good solubility both in organic solvents and in polydimethylsiloxanes (PDMS). MQ resin is also a good reinforcing additive for films.

It is preferred that the particulate filler is included in 1.5 to 20%, preferably 2.5 to 15% by weight of the composition. Further, it is preferred that the total amount of film forming polymer and particulate filler is not more than 25% by weight of the composition.

Without wishing to be bound by theory, the inventors believe that polymers like ethyl cellulose form a uniform occlusive film over the sweat ducts after application and thereby reduce perspiration. Ethyl cellulose in combination with the right solvent and plasticizer forms a robust and adherent film on the skin even in presence of sweat.

Antiperspirant compositions of the present invention may advantageously comprise an additional, non-film forming polymer based antiperspirant active. Whilst this might be a conventional antiperspirant salt comprising Al and/or Zr, such as aluminium chlorohydrate or aluminium-zirconium chlorohydrate optionally complexed with glycine, it is preferred that any additional antiperspirant active is not of this type.

The anti-perspirant active can be applied cosmetically and topically to the skin. In the method of the invention, the composition is wiped across the surface of the skin, depositing a fraction of the composition as it passes. The composition of the invention may be delivered through a stick, in a gel form, using a roll on or through a spray/ aerosol. The preferred form of delivering the present invention is a spray / aerosol.

### Aerosol compositions

The antiperspirant composition may be delivered through an aerosol composition which may comprise a propellant in addition to the other ingredients described hereinabove. Commonly, the propellant is employed in a weight ratio to the base formulation of from 95:5 to 5:95. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; i) low boiling point gasses liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative A suitable propellant for use in the present invention is diethanol amine.

When the composition of the invention is delivered in a roll-on, a firm solid or a stick format, the topically acceptable carrier comprises a hydrophobic carrier or an aqueous carrier. The hydrophobic carrier in such cases may comprise a silicone compound, low boiling alcohol or a wax. When the composition comprises a propellant it is delivered as an aerosol.

### Stick or soft solid compositions

Many different materials have been proposed as gellant for a continuous oil phase, including waxes, small molecule gelling agents and polymers. They each have their advantages and of them, one of the most popular class of gellant has comprised waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although wax fatty alcohols have been employed as gellant for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

### Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

The composition of the present invention can comprise a wide range of other optional components. The CTFA Personal care Ingredient Handbook, Second Edition, 1992, describes a wide variety of nonlimiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, conditioners, exfoliating agents, pH adjusters, preservatives, natural extracts, essential oils, skin sensates, skin soothing agents, and skin healing agents.

The present invention also provides for a method of reducing perspiration comprising the step of applying the composition of the first aspect on to the desired skin surface. The skin surface could be any topical surface which is prone to sweating especially the axilla i.e. the underarm portion of the human body. The method is non-therapeutic

The invention will now be demonstrated with the help of the following examples.

### Examples

The following compositions as shown in Table - 1 were prepared.

The compositions were tested for permeance of the membrane on which the composition is coated, as described below:
A Membrane Permeance Cell -Sweat-o-meter, developed in-house, was used to carry out the experiment. A schematic of the apparatus is shown in Figure - 1.

As shown in Figure -1, a membrane made of PCTE (polycarbonate membrane) of 10 micron pore size and 47 mm diameter procured as PCTF10047100 from Sterlitech was placed in between the membrane holder (3) separating the water reservoir (2) and collection chamber (1). The membrane is coated with the composition being tested. The water reservoir (2) is filled with water and a negative pressure is applied using the vacuum pump (6). The suction pressure is controlled using valve (4) and the suction pressure is measured using pressure gauge (5). At the desired suction pressure, the water from the reservoir passes through the membrane and is collected in the collection chamber (1) which is calibrated. The volume of water collected with time is noted.

70 g of distilled water is poured into the water reservoir. The valve is opened to the desired pressure. The experiment is carried out for a sufficient time to generate a mass-time profile over a set interval e.g. to record the flow across the membrane between 10 and 60 g (50 g total). The data on the permeance is summarized in Table - 1.

The sensorial aspects of the composition on application to skin was measured as given below:
Sensory evaluation was done on visual basis considering parameters such as opacity of film formed after the solvent has evaporated, flexibility, tackiness, and defects/wrinkles formed.

A set of at least 5 respondents were asked to evaluate the films on above mentioned parameters on a scale of 1 to 5, with 5 being more acceptable and 1 the least. An ideal film should not be opaque, flexible enough to feel comfortable to skin, non-tacky, and homogeneous.

The data on the permeance and the sensory score are summarized in the Table below:

**Table -1**

| **Example** | **Composition** | **Flow rate (g/s)** | **Sensory** |
|---|---|---|---|
| A | 10% Ethyl cellulose + rest Ethanol | 0.64 | 2 |
| 1 | 10% Ethyl cellulose+ 5% PPG1200+rest Ethanol | 0.81 | 4 |
| 2 | 10% Ethyl cellulose + 5% PPG1200 + rest Dowanol | 1.30 | 5 |

It is preferred that the desirable composition has a permeance (low flow rate) of less than 3 g/s, preferably less than 2 g/s, more preferably less than 1.5 g/s. It is also preferred that the sensory score is at least 4 or 5. The data in the Table - 1 above indicates that the compositions as per the invention Examples 1 and 2 provides for the desired permeance and sensory value.

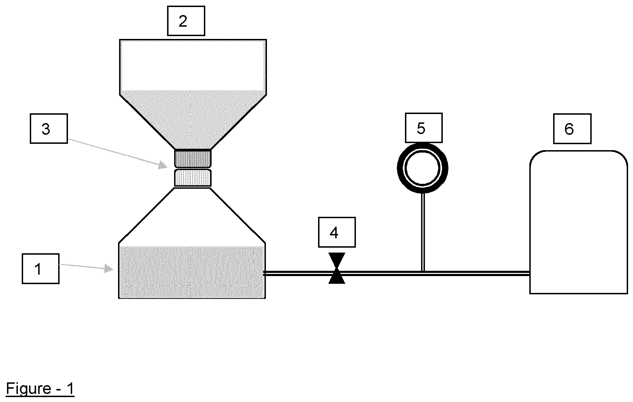

## Claims

1. An anti-perspirant composition comprising:
(i) a film forming polymer selected from modified cellulose having a solubility in water of less than 1 wt%;
(ii) an organic solvent having a Hansen Solubility Parameter in the range of 17 to 28 MPa^{0.5}; and
(iii) a plasticizer;
wherein the modified cellulose is a C1 to C7 alkyl cellulose; and
wherein the plasticizer is selected from one or more of triethyl citrate, polypropylene oxide (with Tg lower than 25°C), glycerol monostearate, stearic acid, cetyl alcohol, triethyl phosphate, or mineral oil.

2. A composition as claimed in claim 1 wherein the modified cellulose is C1 to C4 alkyl cellulose preferably ethyl cellulose.

3. A composition as claimed in claim 2 wherein the ethyl cellulose has a degree of substitution (DS) in the range of 1.7 to 2.8.

4. A composition as claimed in any one of the preceding claims the Hansen partial solubility parameters of the organic solvent are in the ranges: non-polar (δd) from 16.6 to 17.3, polar (δp) from 6.6 to 8.3, and hydrogen bond (δh) from 8.5 to 10.0.

5. A composition as claimed in any one of the preceding claims wherein the organic solvent is chosen from ethanol, toluene, glycol ethers, or isododecane.

6. A composition as claimed in any one of the preceding claims wherein the film forming polymer is included in 1.5 to 25 % by weight of the composition.

7. A composition as claimed in any one of the preceding claims wherein the solvent is included in 50 % to 97 % by weight of the composition.

8. A composition as claimed in any one of the preceding claims wherein the plasticizer is included in 1.5 % to 10% by weight of the composition.

9. A composition as claimed in any one of the preceding claims wherein the total amount of film forming polymer and plasticizer is not more than 25% by weight of the composition.

10. A composition as claimed in any one of the preceding claims additionally comprising a particulate filler selected from MQ resin, silica or clays.

11. A composition as claimed in claim 10 wherein the particulate filler is included in 1.5 to 20%, by weight of the composition.

12. A composition as claimed in any one of the preceding claims wherein the polypropylene oxide is selected from PPG P1200 or PPG P2000.

13. A non-therapeutic method of reducing perspiration comprising the step of applying a composition as claimed in any one of the preceding claims on to the desired skin surface.

## Patentansprüche

1. Schweißhemmende Zusammensetzung, umfassend:
(i) ein filmbildendes Polymer, ausgewählt unter modifizierter Cellulose mit einer Löslichkeit in Wasser von weniger als 1 Gew.-%;
(ii) ein organisches Lösungsmittel mit einem Hansen-Löslichkeitsparameter in dem Bereich von 17 bis 28 MPa^{0,5}; und
(iii) einen Weichmacher;
wobei die modifizierte Cellulose eine C1- bis C7-Alkylcellulose ist; und
wobei der Weichmacher aus einem oder mehreren unter Triethylcitrat, Polypropylenoxid (mit Tg unter 25°C), Glycerolmonostearat, Stearinsäure, Cetylalkohol, Triethylphosphat oder Mineralöl ausgewählt ist.

2. Zusammensetzung, wie im Anspruch 1 beansprucht, wobei die modifizierte Cellulose C1- bis C4-Alkylcellulose, vorzugsweise Ethylcellulose, ist.

3. Zusammensetzung, wie im Anspruch 2 beansprucht, wobei die Ethylcellulose einen Substitutionsgrad (DS) in dem Bereich von 1,7 bis 2,8 aufweist.

4. Zusammensetzung, wie in irgendeinem der vorhergehenden Ansprüche beansprucht, wobei der Hansen-Teillöslichkeitsparameter des organischen Lösungsmittels in den Bereichen liegen: unpolar (δd) von 16,6 bis 17,3, polar (δp) von 6,6 bis 8,3 und Wasserstoffbindung (δh) von 8,5 bis 10,0.

5. Zusammensetzung, wie in irgendeinem der vorhergehenden Ansprüche beansprucht, wobei das organische Lösungsmittel unter Ethanol, Toluol, Glycolethern oder Isododecan ausgewählt ist.

6. Zusammensetzung, wie in irgendeinem der vorhergehenden Ansprüche beansprucht, wobei das filmbildende Polymer mit 1,5 bis 25 Gewichts-% der Zusammensetzung eingeschlossen ist.

7. Zusammensetzung, wie in irgendeinem der vorhergehenden Ansprüche beansprucht, wobei das Lösungsmittel mit 50 bis 97 Gewichts-% der Zusammensetzung eingeschlossen ist.

8. Zusammensetzung, wie in irgendeinem der vorhergehenden Ansprüche beansprucht, wobei der Weichmacher mit 1,5 bis 10 Gewichts-% der Zusammensetzung eingeschlossen ist.

9. Zusammensetzung, wie in irgendeinem der vorhergehenden Ansprüche beansprucht, wobei die Gesamtmenge des filmbildenden Polymers und des Weichmachers nicht mehr als 25 Gewichts-% der Zusammensetzung beträgt.

10. Zusammensetzung, wie in irgendeinem der vorhergehenden Ansprüche beansprucht, zusätzlich umfassend einen teilchenförmigen Füllstoff, ausgewählt unter MQ-Harz, Siliziumdioxid oder Tonen.

11. Zusammensetzung, wie im Anspruch 10 beansprucht, wobei der teilchenförmige Füllstoff mit 1,5 bis 20 Gewichts-% der Zusammensetzung eingeschlossen ist.

12. Zusammensetzung, wie in irgendeinem der vorhergehenden Ansprüche beansprucht, wobei das Polypropylenoxid unter PPG P1200 oder PPG P2000 ausgewählt ist.

13. Nicht-therapeutisches Verfahren zum Vermindern der Schweißbildung, umfassend den Schritt des Auftragens einer Zusammensetzung, wie in irgendeinem der vorhergehenden Ansprüche beansprucht, auf die gewünschte Hautoberfläche.

## Revendications

1. Composition d'anti-perspirant comprenant :
(i) un polymère formant un film choisi parmi de la cellulose modifiée ayant une solubilité dans l'eau inférieure à 1 % en masse ;
(ii) un solvant organique ayant un paramètre de solubilité Hansen dans l'intervalle de 17 à 28 MPa^{0,5} ; et
(iii) un plastifiant ;
dans laquelle la cellulose modifiée est une alkylcellulose en C1 à C7 ; et
dans laquelle le plastifiant est choisi parmi un ou plusieurs de citrate de triéthyle, poly(oxyde de propylène) (avec une Tg inférieure à 25°C), monostéarate de glycérol, acide stéarique, alcool cétylique, phosphate de triéthyle, ou huile minérale.

2. Composition selon la revendication 1, dans laquelle la cellulose modifiée est une alkylcellulose en C1 à C4 de préférence l'éthylcellulose.

3. Composition selon la revendication 2, dans laquelle l'éthylcellulose présente un degré de substitution (DS) dans l'intervalle de 1,7 à 2,8.

4. Composition selon l'une quelconque des revendications précédentes, les paramètres de solubilité partielle Hansen du solvant organique se trouvent dans les intervalles : non-polaire (δd) de 16,6 à 17,3, polaire (δp) de 6,6 à 8,3, et liaison hydrogène (δh) de 8,5 à 10,0.

5. Composition selon l'une quelconque des revendications précédentes, dans laquelle le solvant organique est choisi parmi l'éthanol, le toluène, des glycoléthers, ou l'isododécane.

6. Composition selon l'une quelconque des revendications précédentes, dans laquelle le polymère formant un film est inclus dans de 1,5 à 25 % en masse de la composition.

7. Composition selon l'une quelconque des revendications précédentes, dans laquelle le solvant est inclus dans de 50 % à 97 % en masse de la composition.

8. Composition selon l'une quelconque des revendications précédentes, dans laquelle le plastifiant est inclus dans de 1,5 % à 10 % en masse de la composition.

9. Composition selon l'une quelconque des revendications précédentes, dans laquelle la quantité totale de polymère formant un film et de plastifiant est d'au plus 25 % en masse de la composition.

10. Composition selon l'une quelconque des revendications précédentes comprenant de plus une charge particulaire choisie parmi une résine MQ, de la silice ou des argiles.

11. Composition selon la revendication 10, dans laquelle la charge particulaire est incluse dans de 1,5 à 20 %, en masse de la composition.

12. Composition selon l'une quelconque des revendications précédentes, dans laquelle le poly(oxyde de propylène) est choisi parmi PPG P1200 ou PPG P2000.

13. Procédé non-thérapeutique de réduction de la transpiration comprenant l'étape d'application d'une composition selon l'une quelconque des revendications précédentes sur la surface de peau souhaitée.
